# EUROPÄISCHE PATENTSCHRIFT

(11) **EP 0 697 027 B1**
(45) Veröffentlichungstag und Bekanntmachung des Hinweises auf die Patenterteilung: **02.07.1997**
(21) Anmeldenummer: 94915140.1
(22) Anmeldetag: 28.04.1994
(51) Int. Cl.: C08G 73/10

(54) **TETRAAROXYPERYLEN-3,4,9,10-TETRACARBONSÄUREPOLYIMIDE**
TETRAAROXYPERYLENE-3,4,9,10-TETRACARBOXYLIC ACID POLYIMIDES
POLYIMIDES D'ACIDE TETRAAROXYPERYLENE-3,4,9,10-TETRACARBOXYLIQUE

(30) Priorität: 04.05.1993 DE 4314622
(43) Veröffentlichungstag der Anmeldung: 21.02.1996
(73) Patentinhaber: Max-Planck-Gesellschaft zur Förderung der Wissenschaften e.V., 80539 München (DE)
(72) Erfinder: MUELLEN, Klaus, D-50939 Köln (DE); DOTCHEVA, Dobrinka, Todorova, 1407 Sofia (BG); KLAPPER, Markus, D-65428 Rüsselsheim (DE)
(74) Vertreter: Karg, Jochen
(86) Internationale Anmeldenummer: EP9401345
(87) Internationale Veröffentlichungsnummer: WO9425504

(56) Entgegenhaltungen:
- EP-A- 0 445 577
- PATENT ABSTRACTS OF JAPAN vol. 12, no. 353 (C-530)(3200) 21. September 1988 & JP,A,63 110 219 (TOSOH CORPORATION)
- PATENT ABSTRACTS OF JAPAN vol. 16, no. 244 (C-947)(5287) 4. Juni 1992 & JP,A,04 053 894 (NIHON KAGAKU HATSUKOU K.K.)
- PATENT ABSTRACTS OF JAPAN vol. 12, no. 353 (C-530)(3200) 21. September 1988 & JP,A,63 110 219
- PATENT ABSTRACTS OF JAPAN vol. 16, no. 244 (C-947)(5287) 4. Juni 1992 & JP,A,04 053 894

## Beschreibung

Die vorliegende Erfindung betrifft neue Tetraaroxyperylen-3,4,9,10-tetracarbonsäurepolyimide der allgemeinen Formel I 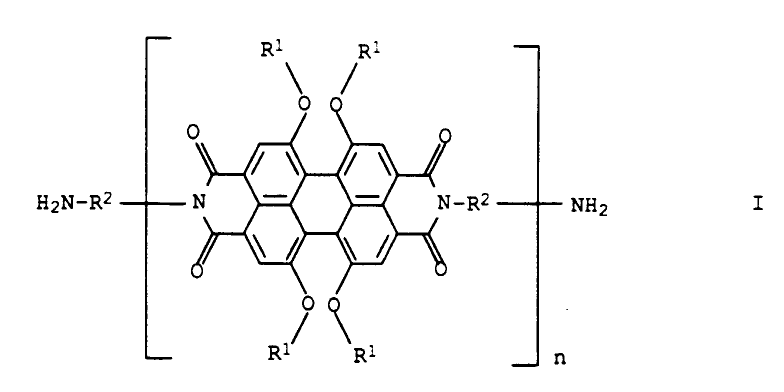 in der die Variablen folgende Bedeutung haben:
- R¹: gleiche oder verschiedene Arylreste, die durch Cyano, Nitro, Halogen, C₁-C₁₈-Alkoxy, C₅-C₇-Cycloalkyl und/oder C₁-C₁₈-Alkyl substituiert sein können und jeweils bis zu 24 C-Atome enthalten können;
- R²: C₂-C₃₀-Alkylengruppen, deren Kohlenstoffkette durch 1 bis 10 Sauerstoffatome in Etherfunktion oder eine Phenylen- oder Cyclohexylengruppe unterbrochen sein kann, oder gegebenenfalls durch C₁-C₁₀-Alkylen oder Sauerstoff verbrückte C₆-C₃₀-Arylen- oder Cyclohexylenreste;
- n: 2 bis 100,
sowie die Herstellung dieser Polyimide und ihre Verwendung in Farbstofflasern, Lichtsammelsystemen, Druckfarben und Anstrichmitteln und zur Einfärbung von Kunststoffen.

Weiterhin betrifft die Erfindung neue Tetraaroxyperylen-3,4,9,10-tetracarbonsäuredianhydride der allgemeinen Formel III 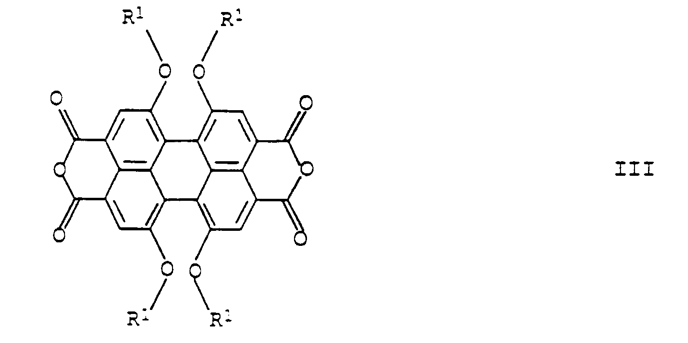 und die entsprechenden Tetraaroxyperylen-3,4,9,10-tetracarbonsäuren (IIIa) als Zwischenprodukte für die Herstellung der Polyimide I.

Für Fluoreszenzfarbstoffe gibt es bekanntermaßen vielfältige Anwendungsmöglichkeiten; sie werden beispielsweise zur flächenmäßigen Konzentrierung von Licht in Lichtsammelsystemen, zur Herstellung von Farbstofflasern und Tagesleuchtfarben sowie auch allgemein zur Einfärbung von Druckfarben, Anstrichmitteln und Polymeren eingesetzt.

Hierfür müssen die Farbstoffe eine Reihe von Eigenschaften aufweisen, die insbesondere für Lichtsammelsysteme hoch sind. Sie müssen hohe Fluoreszenz, einen breiten Absorptionsbereich, gute Trennung von Absorptions- und Emissionsbanden im Anwendungsmedium, hohe Lichtechtheit und gute Löslichkeit und gleichzeitig geringe Neigung zur Migration im Anwendungsmedium aufweisen.

Diese Anforderungen sind gleichzeitig nur schwer zu erfüllen. Das gilt auch für die in der EP-A-227 980 und der JP-A-53 894/1992 beschriebenen Fluoreszenzfarbstoffe auf der Basis von monomeren, vierfach substituierten Perylen-3,4,9,10-tetracarbonsäurediimiden, die hinsichtlich ihrer Migrationsbeständigkeit teilweise nicht zufriedenstellend sind.

Aus der JP-A-110 219/1988 und der EP-A-445 577 sind unsubstituierte Perylen-3,4,9,10-tetracarbonsäurepolyimide bekannt, die jedoch nicht als Fluoreszenzfarbstoffe verwendet werden.

Der Erfindung lag die Aufgabe zugrunde, Fluoreszenzfarbstoffe bereitzustellen, die dem geforderten Eigenschaftsprofil besonders nahe kommen.

Demgemäß wurden die eingangs definierten Tetraaroxyperylen-3,4,9,10-tetracarbonsäurepolyimide I gefunden.

Weiterhin wurde ein Verfahren zur Herstellung der Polyimide I gefunden, welches dadurch gekennzeichnet ist, daß man
a) ein Tetraaroxyperylen-3,4,9,10-tetracarbonsäurediimid der allgemeinen Formel II 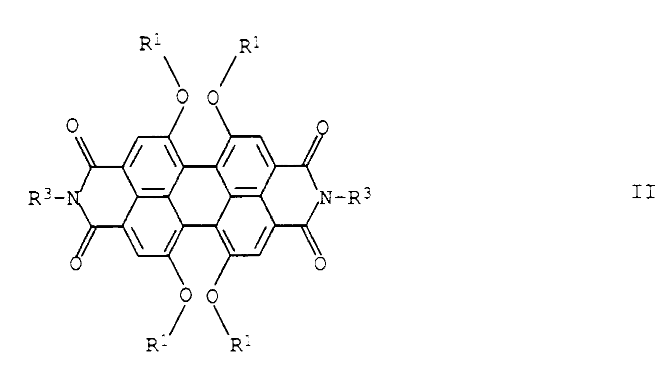 in der die Reste R³ C₁-C₃₀-Alkylreste, deren Kohlenstoffkette durch 1 bis 10 Sauerstoffatome in Etherfunktion unterbrochen sein kann, C₅-C₇-Cycloalkyl- oder Phenylreste, die jeweils durch bis zu 2 C₁-C₄-Alkylgruppen substituiert sein können, bedeuten,
   mit einer Base in einem polaren, protischen Lösungsmittel behandelt und
b) das in Schritt a) erhaltene Tetraaroxyperylen-3,4,9,10-tetracarbonsäuredianhydrid der allgemeinen Formel III 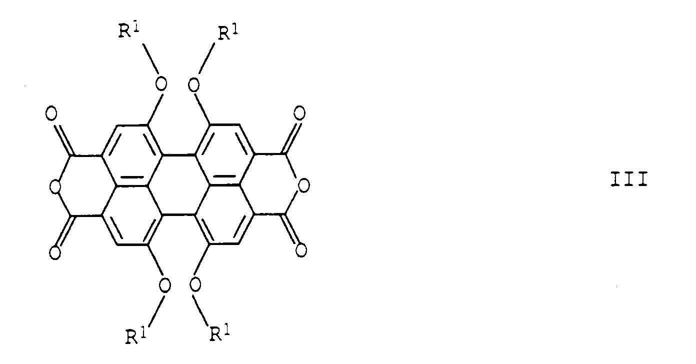 mit einem Diamin der allgemeinen Formel IV

   H₂N-R²-NH₂ IV

   in Gegenwart eines polaren Lösungsmittels zunächst unter saurer und dann unter basischer Katalyse bei 170 bis 200°C polykondensiert.

Außerdem wurde die Verwendung der Polyimide I zur Herstellung von Farbstofflasern und Lichtsammelsystemen und zur Pigmentierung von Druckfarben, Anstrichmitteln und Kunststoffen gefunden.

Nicht zuletzt wurden die Tetraaroxyperylen-3,4,9,10-tetracarbonsäuredianhydride III und die entsprechenden Tetraaroxyperylen-3,4,9,10-tetracarbonsäuren (IIIa) als Zwischenprodukte für die Herstellung der Polyimide I gefunden.

Geeignete Reste R¹ sind dabei Arylreste, wie Naphthyl, Anthryl und besonders Phenyl. Die Arylreste können durch Cyano, Nitro, Halogen, insbesondere Chlor, C₁-C₁₈-Alkoxy, C₅-C₇-Cycloalkyl, vor allem Cyclohexyl, und/oder C₁-C₁₈-Alkyl substituiert sein und enthalten jeweils in der Regel bis zu 24 C-Atome. Die Reste R¹ können gleich oder verschieden sein, sind jedoch bevorzugt gleich.

Beispiele für die als Substituenten geeigneten C₁-C₁₈-Alkoxy- und Alkylgruppen sind:
- Methoxy, Ethoxy, Propoxy, Isopropoxy, Butoxy, Isobutoxy, sec.-Butoxy, tert.-Butoxy, Pentyloxy, Isopentyloxy, Neopentyloxy, Hexyloxy, Heptyloxy, Octyloxy, 2-Ethylhexyloxy, Nonyloxy, Decyloxy, Undecyloxy, Dodecyloxy, Tridecyloxy, Tetradecyloxy, Pentadecyloxy, Hexadecyloxy, Heptadecyloxy und Octadecyloxy sowie weitere verzweigte Reste dieser Art;
- Methyl, Ethyl, Propyl, Isopropyl, Butyl, Isobutyl, sec.-Butyl, tert.-Butyl, Pentyl, Isopentyl, Neopentyl, tert.-Pentyl, Hexyl, 2-Methylpentyl, Heptyl, Octyl, 2-Ethylhexyl, Nonyl, Decyl, Undecyl, Dodecyl, Tridecyl, Tetradecyl, Pentadecyl, Hexadecyl, Heptadecyl und Octadecyl sowie weitere verzweigte Reste dieser Art.

Beispiele für bevorzugte substituierte Arylreste sind 4-tert.-Butylphenyl, 2-Cyclohexyl-4-methylphenyl, 2- und 4-Cyclohexylphenyl, 4-Hexyloxyphenyl, 2-Phenylphenyl, 4-Cyanophenyl-O-nitrophenyl, Benzylphenyl und 2-Benzyl-4-chlorphenyl.

Bei den Gruppen R² handelt es sich vorzugsweise um aliphatische, cycloaliphatische oder aromatische Gruppen.

Geeignete Alkylengruppen enthalten in der Regel 2 bis 30, bevorzugt 3 bis 10 C-Atome. Beispielsweise seien genannt: Ethylen, 1,2- und 1,3-Propylen, 1,2-, 1,3-, 1,4- und 2,3-Butylen, 1,2-, 1,3-, 1,4-, 1,5-, 2,3- und 2,4-Pentylen, Hexamethylen, Heptamethylen, Octamethylen, Nonamethylen und Decamethylen sowie weitere verzweigte Reste dieser Art.

Zusätzlich kann die Kohlenstoffkette dieser Gruppen noch durch 1 bis 10 Sauerstoffatome in Etherfunktion oder Phenylen oder Cyclohexylen unterbrochen sein. Als Beispiele seien genannt (dabei bedeuten Ph= Phenylen, C₆H₄= Cyclohexylen):
-(CH₂)₂-O-(CH₂)₂-, -[(CH₂)₂-O]₂-(CH₂)₂-, -[(CH₂)₂-O]₃-(CH₂)₂-, -(CH₂)₃-O-(CH₂)₄-O-(CH₂)₃-, -(CH₂)₃-O-[(CH₂)₂-O]₂-(CH₂)₃-, -CH₂-Ph-CH₂- und -CH₂-C₆H₄-CH₂-.

Geeignete cyclische Gruppen R² sind insbesondere Cyclohexylen und Phenylen, aber auch Naphthylen. Insbesondere können auch zwei Arylen- oder Cyclohexylenreste über Sauerstoff oder C₁-C₁₀-Alkylen verbrückt sein. Beispiele für diese Gruppierungen sind (dabei bedeutet m= 1 bis 10):

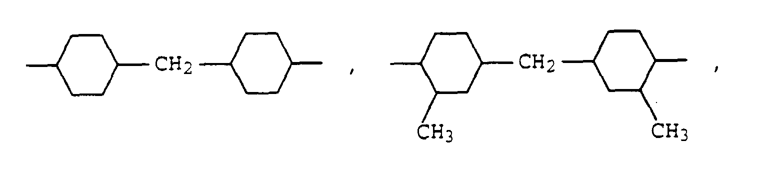

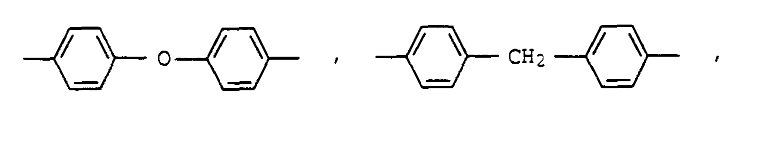

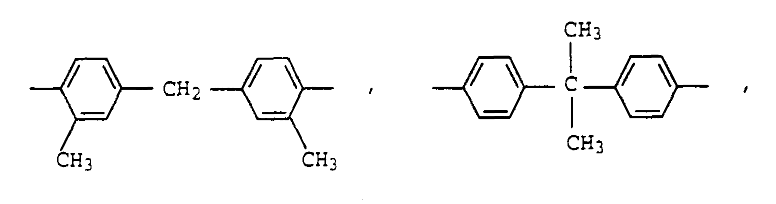

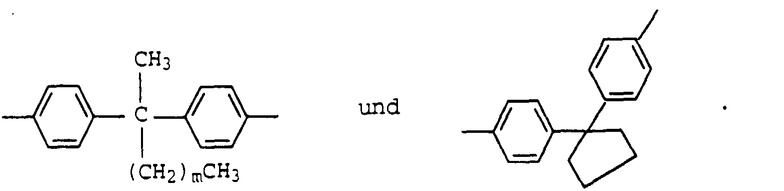

Die Variable n bedeutet schließlich einen Mittelwert für die Anzahl der im Polyimid I enthaltenen Perylendiimideinheiten und beträgt in der Regel 2 bis 100, bevorzugt 10 bis 50 und besonders bevorzugt 20 bis 40.

Die erfindungsgemäßen Polyimide I zeichnen sich insgesamt durch hervorragende Eigenschaften aus: Sie weisen hohe Lichtechtheit, hohe thermische Stabilität, hohe Fluoreszenz und einen breiten Absorptionsbereich auf. Sie sind trotz ihrer Molekülgröße sehr gut löslich und zeigen gleichzeitig nur geringe Migrationstendenz im jeweiligen Anwendungsmedium. Zudem sind sie leicht filmbildend und ziehen als homogener Film auf Glas oder Kunststoff auf.

Das erfindungsgemäße Verfahren zur Herstellung der Polyimide I läuft zweistufig über die neuen Tetraaroxyperylen-3,4,9,10-tetracarbonsäuredianhydride III bzw. die entsprechenden Säuren (IIIa) als Zwischenprodukte ab.

Als Edukte bei der in Schritt a) erfolgenden Synthese der Anhydride III werden die oben definierten Tetraaroxyperylen-3,4,9,10-tetracarbonsäurediimide II eingesetzt.

Geeignete Reste R³ am Imidstickstoffatom- sind dabei C₁-C₃₀-Alkylreste, vorzugsweise C₁-C₁₈-Alkylreste, wie sie bei R¹ genannt sind, C₅-C₇-Cycloalkylreste, vor allem Cyclohexylreste, und Phenylreste.

Die Kohlenstoffkette der Alkylreste kann durch 1 bis 10 Sauerstoffatome in Etherfunktion unterbrochen sein. Beispiele hierfür sind: 2-Methoxy-, 2-Ethoxy-, 2-Propoxy-, 2-Isopropoxy- und 2-Butoxyethyl, 2- und 3-Methoxypropyl, 2- und 3-Ethoxypropyl, 2- und 4-Ethoxybutyl, 2- und 4-Isopropoxybutyl, 5-Ethoxypentyl, 6-Methoxyhexyl, 4-Oxa-6-ethyldecyl, 3,6-Dioxaheptyl, 3,6-Dioxaoctyl, 3,6-Dioxadecyl, 3,6,9-Trioxadecyl und 3,6,8-Trioxaundecyl.

Die Cycloalkyl- und Phenylreste können durch bis zu 2 C₁-C₄-Alkylgruppen, wie sie ebenfalls oben genannt sind, substituiert sein.

Die Perylimide II sind aus der EP-A-227 980 bekannt und können, wie dort beschrieben, durch Umsetzung der Tetrachlorperylimide mit den entsprechenden Arylaten hergestellt werden.

In Schritt a) des erfindungsgemäßen Verfahrens wird ein Perylimid II durch Einwirkung einer Base in Gegenwart eines polaren, protischen Lösungsmittels in das entsprechende Anhydrid III überführt. Die Säure (IIIa) selbst ist nicht beständig und wandelt sich praktisch sofort in das Anhydrid um.

Als Basen sind dabei vor allem anorganische Basen wie Natriumhydroxid und Kaliumhydroxid geeignet.

Wie für derartige Reaktionen üblich, wird die Base im Überschuß, d.h. in der Regel in Mengen von 1 bis 10 mol pro 10 mmol, eingesetzt.

Als polare, protische Lösungsmittel kommen insbesondere C₁-C₁₀-Alkanole wie Propanol, Isopropanol, n-Butanol, n-Hexanol, n-Decanol, vorzugsweise tert.-Butanol in Frage. Zweckmäßigerweise wird noch eine geringe Menge Wasser, im allgemeinen 0,1 bis 0,4 mol pro mmol II zugefügt, um die Abtrennung des Produkts zu erleichtern.

Auch die Menge Lösungsmittel ist an sich unkritisch. Übliche Mengen sind 0,5 bis 1,2 mol pro mmol II.

Zweckmäßigerweise geht man in Schritt a) des erfindungsgemäßen Verfahrens so vor, daß man die Mischung aus Perylendiimid II, Base und Lösungsmittel mehrere h, in der Regel 10 bis 48 h, unter Rückfluß, d.h. etwa auf 80 bis 140°C, erhitzt. Nach Abkühlen auf Raumtemperatur kann dann die organische Phase abgetrennt werden. Die Isolation des Anhydrids III kann in üblicher Weise durch Ausfällen mit einer Säure, Abfiltrieren, Waschen und Trocknen erfolgen.

In Schritt b) des erfindungsgemäßen Verfahrens werden das in a) erhaltene Anhydrid III und ein Diamin IV polymerisiert.

Diese Polykondensation wird bevorzugt in Gegenwart eines polaren Lösungsmittels zunächst unter saurer und anschließend unter basischer Katalyse durchgeführt.

Als polare Lösungsmittel eignen sich die bei der Herstellung von Polyimiden eingesetzten Lösungsmittel. Als bevorzugte Beispiele seien Kresol, Dimethylformamid, Dimethylacetamid, N-Methylpyridon, 1,3-Dimethyl-3,4,5,6-tetrahydropyrimidin-2-on genannt. Das Lösungsmittel wird üblicherweise in Mengen von 0,8 bis 2 mol, vorzugsweise um 1 mol, pro 10 mmol Diamin IV eingesetzt.

Als saurer Katalysator ist z.B. Benzoesäure bevorzugt. Im allgemeinen verwendet man 2 bis 4 mol, insbesondere 2 mol Katalysator pro mol III. Besonders geeignete basische Katalysatoren sind beispielsweise Chinolin und Isochinolin, die üblicherweise ebenfalls in Mengen von etwa 2 bis 4 mol, vorzugsweise 2 mol, pro mol III eingesetzt werden.

Die Reaktionstemperatur beträgt in der Regel 170 bis 200°C.

Zweckmäßigerweise geht man bei der Polykondensation so vor, daß man eine Mischung aus Diamin IV, Lösungsmittel, Anhydrid III und saurem Katalysator zunächst etwa 8 bis 12 h und nach Zugabe des basischen Katalysators üblicherweise weitere 15 bis 24 h auf die Reaktionstemperatur erhitzt. Nach Abkühlen auf Raumtemperatur kann das Polyimid I durch Lösen in Chloroform vom Reaktionsgemisch abgetrennt und durch Zugabe von Methanol wieder ausgefällt werden.

Das erfindungsgemäße Verfahren zeichnet sich durch gute Ausbeuten und hohe Reinheit der erhaltenen Polyimide I aus.

Die Polyimide I eignen sich vorteilhaft für die für Fluoreszenzfarbstoffe üblichen Anwendungszwecke wie die Herstellung von Lichtsammelsystemen und Farbstofflasern, die in der EP-A-227 980 bzw. der DE-A-34 13 418 und der jeweils angegebenen Literatur beschrieben ist. Weiterhin können sie zur Pigmentierung von Druckfarben, Anstrichmitteln, insbesondere auch Tagesleuchtfarben, und Kunststoffen eingesetzt werden.

### Beispiele

Herstellung von polymeren Tetraaroxyperylen-3,4,9,10-tetracarbonsäurediimiden I'
a) Herstellung der Tetraaroxyperylen-3,4,9,10-tetracarbonsäuredianhydride III' 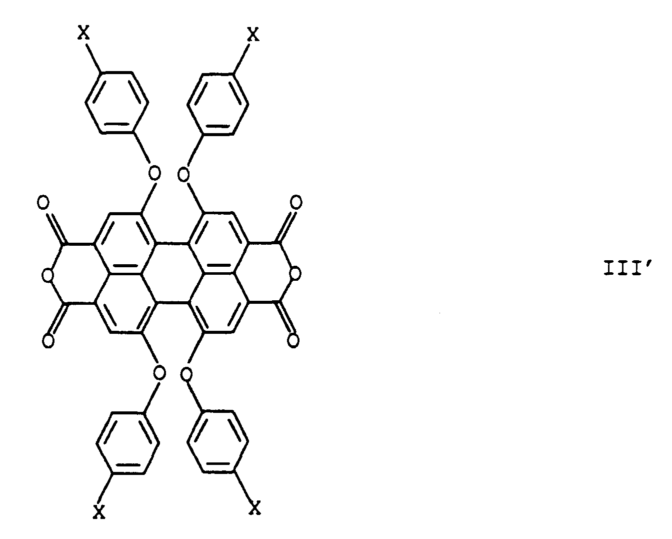 Eine Mischung aus 0,74 mmol eines Perylimids II 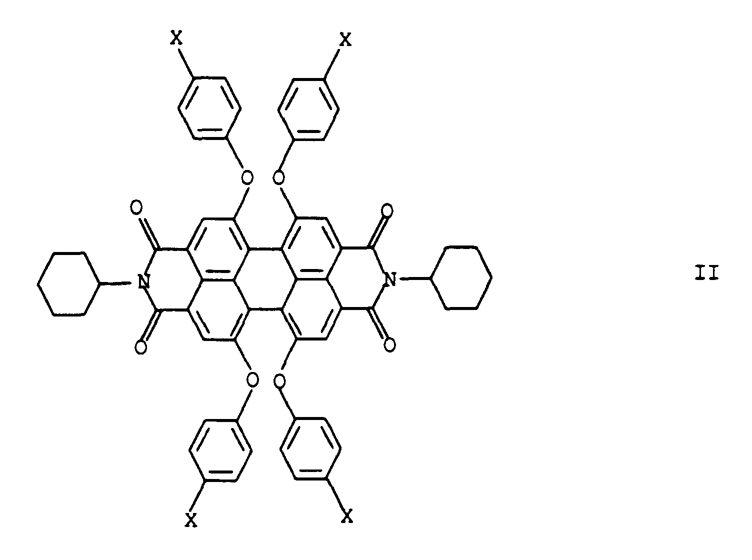 und 6,0 g Kaliumhydroxid, 60 ml tert.-Butanol und 3 ml Wasser wurde 25 h unter Rühren zum Rückfluß erhitzt. Nach Abkühlen auf Raumtemperatur wurde die flüssige organische Phase abgetrennt und mit dem gleichen Volumen 2 N Salzsäure versetzt.

Nach 8 h wurde der Niederschlag abfiltriert, mit Wasser neutral gewaschen und im Vakuum bei 100°C getrocknet. Zur weiteren Reinigung wurde das Produkt erneut in 5 gew.-%iger ethanolischer Natriumhydroxidlösung gelöst und durch Zugabe von 2 N Salzsäure wieder ausgefällt.

Weitere Einzelheiten zu diesen Versuchen und deren Ergebnisse sind in Tabelle 1 aufgeführt.

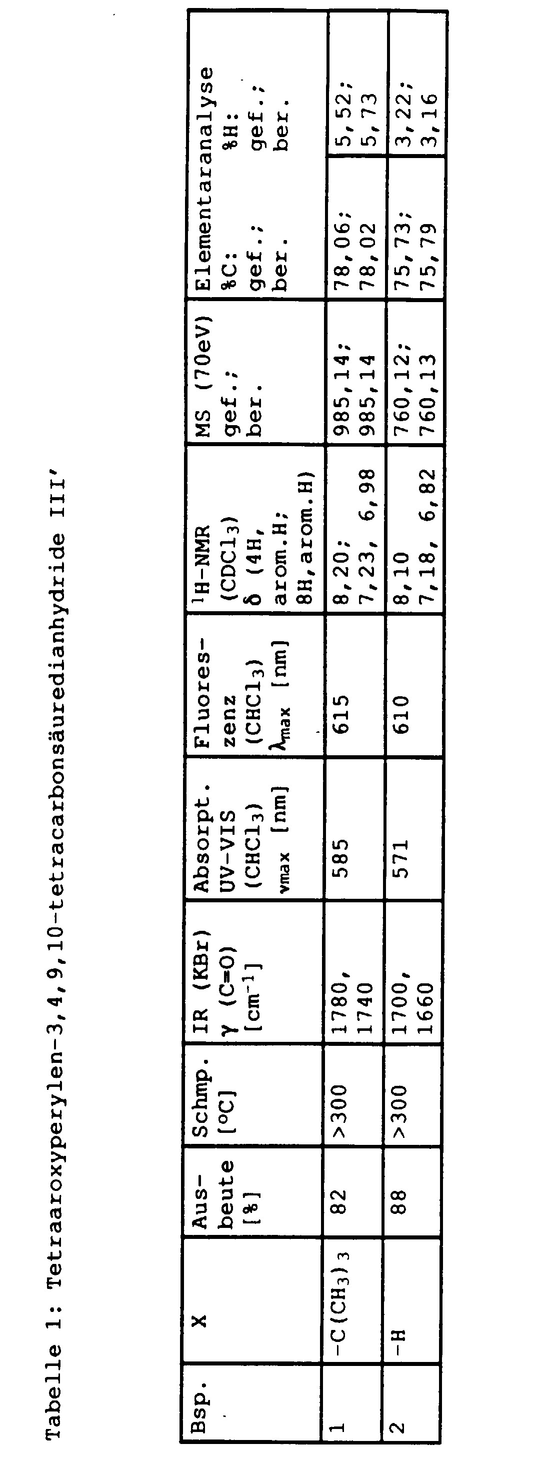

b) Herstellung der polymeren Tetraaroxyperylen-3,4,9,10-tetracarbonsäurediimide I'

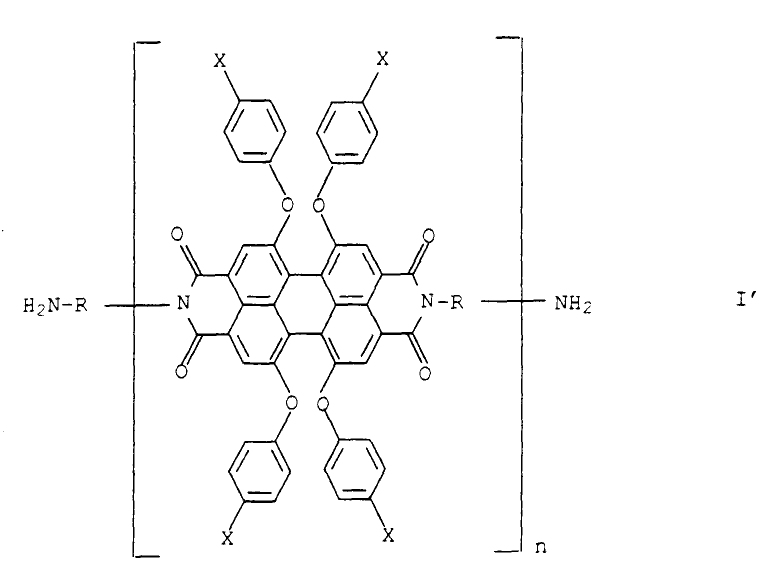

Eine Mischung aus 0,3 mmol eines Diamins IV'

H₂N-R-NH₂ IV'

gelöst in 3 ml m-Kresol, 0,3 mmol eines Anhydrids III' und 0,6 mmol Benzoesäure wurde unter Rühren und Einleiten von Argon auf 190°C erhitzt und 9 h bei dieser Temperatur gehalten. Nach Zusatz von 0,6 mmol Isochinolin, gelöst in 0,3 ml m-Kresol, wurde die Polykondensation weitere 19 h bei 190°C fortgesetzt.

Dann wurde die viskose Reaktionsmasse auf Raumtemperatur abgekühlt, und Chloroform wurde zugegeben. Die erhaltene Polymerlösung wurde filtriert, eingeengt, und das Polyimid I' wurde durch Zugabe von Methanol ausgefällt, rekristallisiert und bei Normaldruck über einen G4-Glasfilter filtriert.

Alle erhaltenen Polyimide I' zeigten die charakteristischen Schwingungen für aromatische Imide in Sechsringen bei 1700 cm⁻¹ und für an zwei aromatische Ringe gebundene Carbonylgruppen bei 1660 cm⁻¹ und waren an Luft und unter Stickstoff bis 350°C thermisch stabil.

Weitere Einzelheiten zu diesen Versuchen und ihre Ergebnisse sind in Tabelle 2 und 3 zusammengestellt.

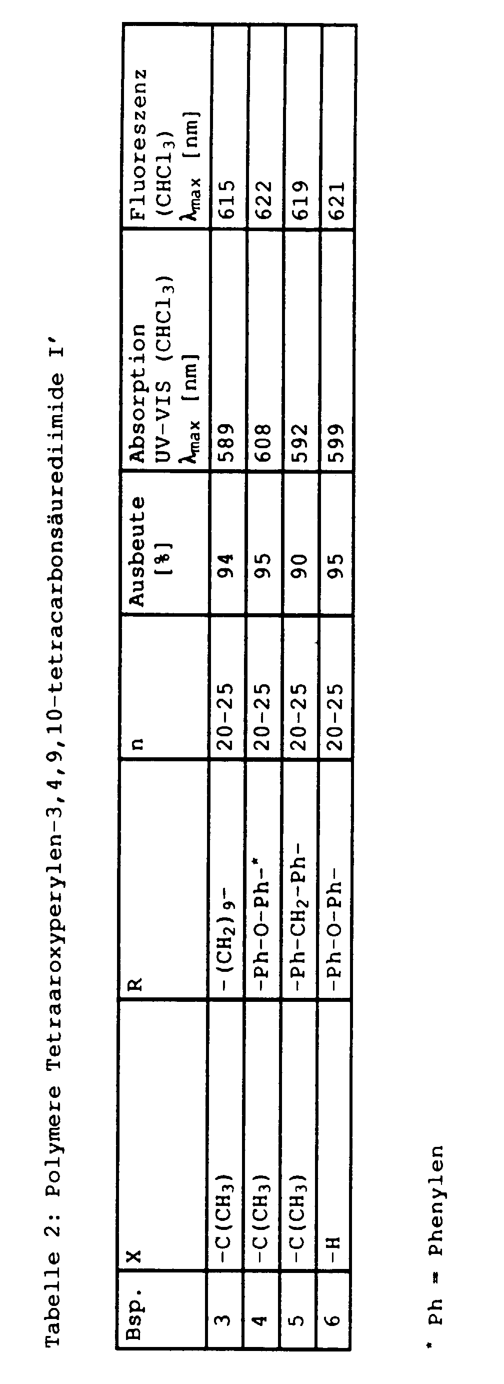

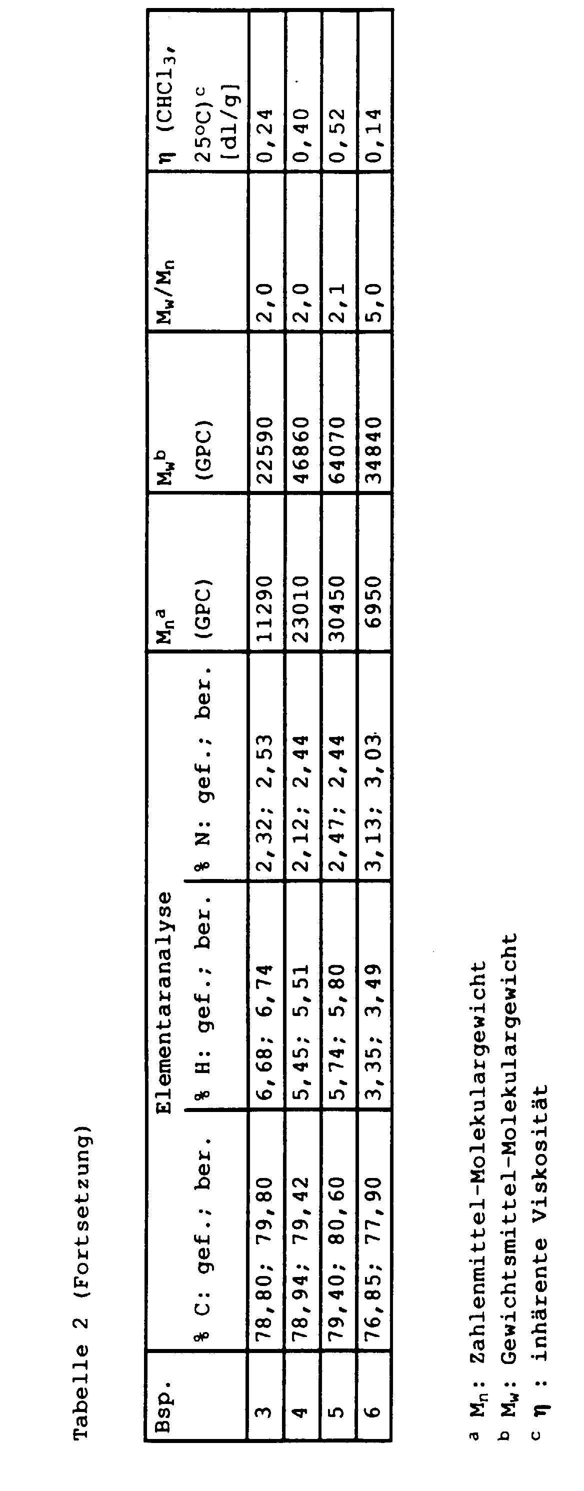

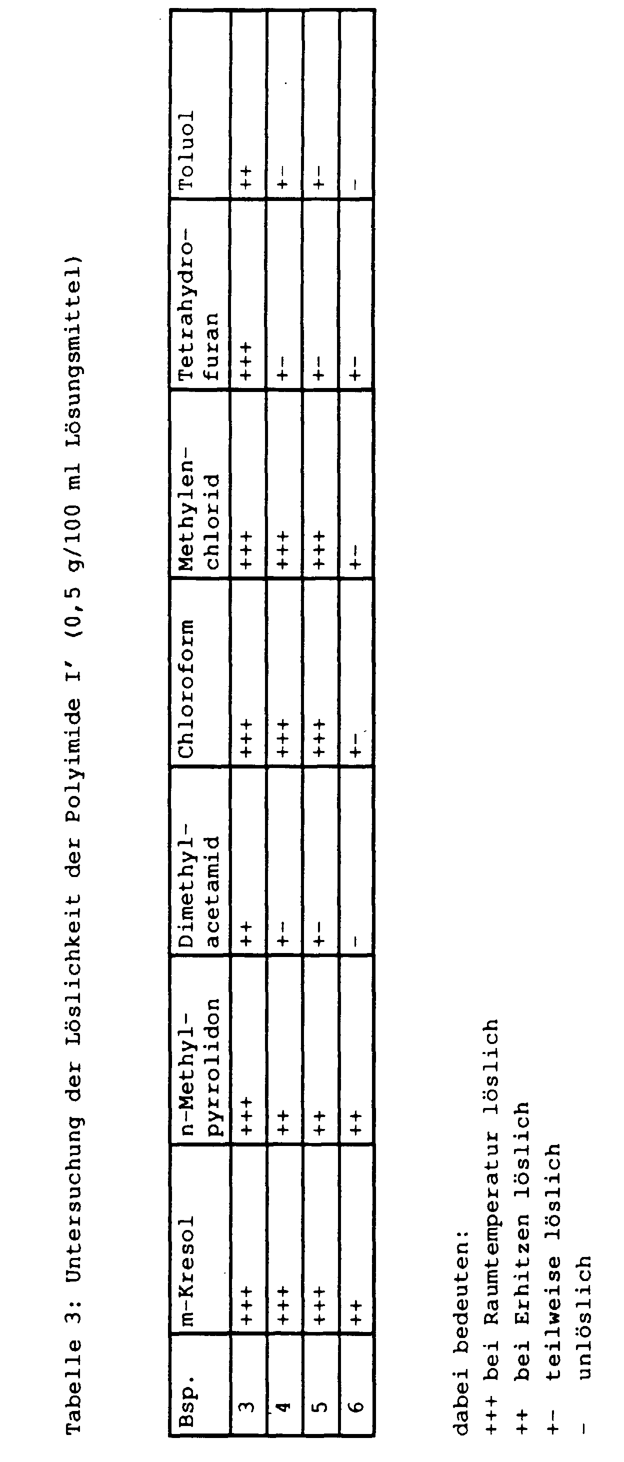

## Patentansprüche

1. Tetraaroxyperylen-3,4,9,10-tetracarbonsäurepolyimide der allgemeinen Formel I 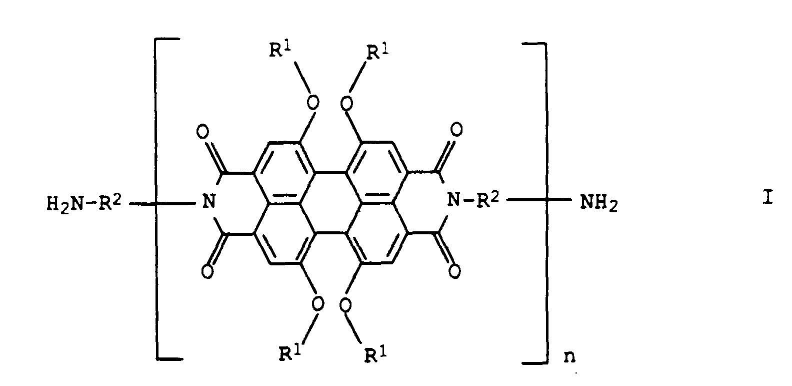 in der die Variablen folgende Bedeutung haben:
R¹ gleiche oder verschiedene Arylreste, die durch Cyano, Nitro, Halogen, C₁-C₁₈-Alkoxy, C₅-C₇-Cycloalkyl und/oder C₁-C₁₈-Alkyl substituiert sein können und jeweils bis zu 24 C-Atome enthalten können;
R² C₂-C₃₀-Alkylengruppen, deren Kohlenstoffkette durch 1 bis 10 Sauerstoffatome in Etherfunktion oder eine Phenylen- oder Cyclohexylengruppe unterbrochen sein kann, oder gegebenenfalls durch C₁-C₁₀-Alkylen oder Sauerstoff verbrückte C₆-C₃₀-Arylen- oder Cyclohexylenreste;
n 2 bis 100.

2. Verfahren zur Herstellung von Tetraaroxyperylen-3,4,9,10- tetracarbonsäurepolyimiden der Formel I gemäß Anspruch 1, dadurch gekennzeichnet, daß man
a) ein Tetraaroxyperylen-3,4,9,10-tetracarbonsäurediimid der allgemeinen Formel II 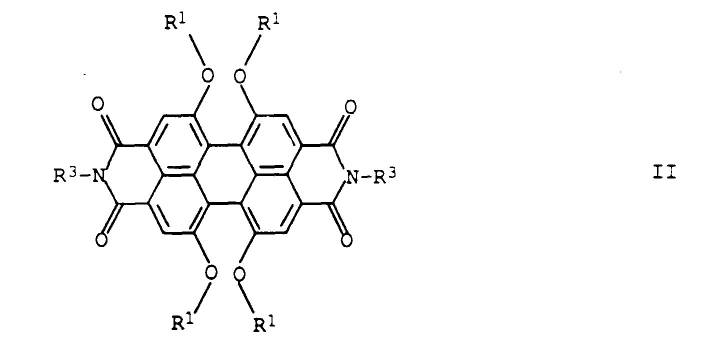 in der die Reste R³ C₁-C₃₀-Alkylreste, deren Kohlenstoffkette durch 1 bis 10 Sauerstoffatome in Etherfunktion unterbrochen sein kann, C₅-C₇-Cycloalkyl- oder Phenylreste, die jeweils durch bis zu 2 C₁-C₄-Alkylgruppen substituiert sein können; bedeuten,
mit einer Base in einem polaren, protischen Lösungsmittel behandelt und
b) das in Schritt a) erhaltene Tetraaroxyperylen-3,4,9,10-tetracarbonsäuredianhydrid der allgemeinen Formel III 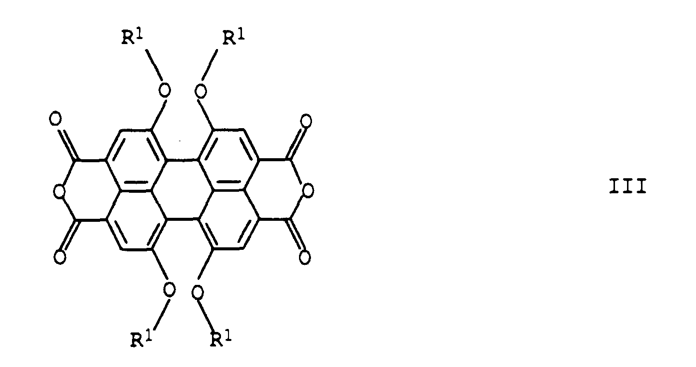 mit einem Diamin der allgemeinen Formel IV
H₂N-R²-NH₂ IV
in Gegenwart eines polaren Lösungsmittels zunächst unter saurer und dann unter basischer Katalyse bei 170 bis 200°C polykondensiert.

3. Verwendung von Tetraaroxyperylen-3,4,9,10-tetracarbonsäurepolyimiden der Formel I gemäß Anspruch 1 zur Herstellung von Farbstofflasern und Lichtsammelsystemen und zur Pigmentierung von Druckfarben, Anstrichmitteln und Kunststoffen.

4. Tetraaroxyperylen-3,4,9,10-tetracarbonsäuredianhydride der allgemeinen Formel III 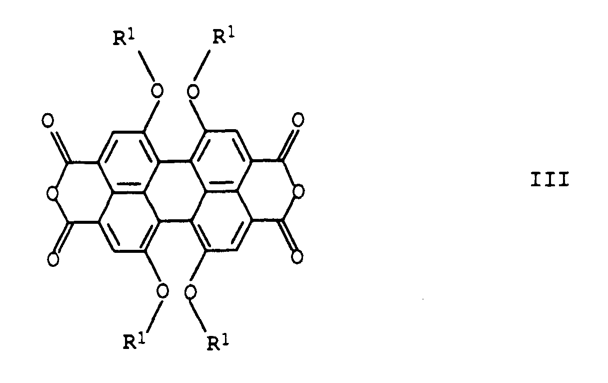 in der die Reste R¹ gleiche oder verschiedene Arylreste bedeuten, die durch Cyano, Nitro, Halogen, C₁-C₁₈-Alkoxy, C₅-C₇-Cycloalkyl und/oder C₁-C₁₈-Alkyl substituiert sein können und jeweils bis zu 24 C-Atome enthalten können,
und die entsprechenden Tetraaroxyperylen-3,4,9,10-tetracarbonsäuren (IIIa).

## Claims

1. Tetraaroxyperylene-3,4,9,10-tetracarboxylic polyimides of the general formula I 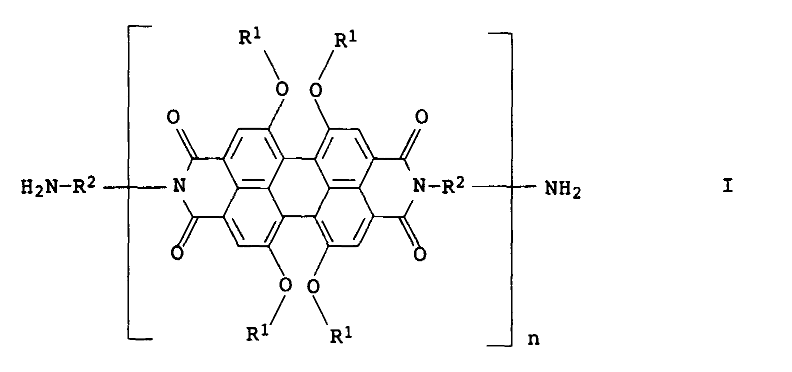 where
R¹ denotes identical or different aryl radicals which may be substituted by cyano, nitro, halogen, C₁-C₁₈-alkoxy, C₅-C₇-cycloalkyl and/or C₁-C₁₈-alkyl and may each contain up to 24 carbon atoms;
R² denotes C₂-C₃₀-alkylene groups whose carbon chain may be interrupted by from 1 to 10 oxygen atoms in ether function or by a phenylene or cyclohexylene group, or optionally C₁-C₁₀-alkylene- or oxygen-bridged C₆-C₃₀-arylene or cyclohexylene radicals;
n is from 2 to 100.

2. A process for preparing tetraaroxyperylene-3,4,9,10-tetracarboxylic polyimides of the formula I as set forth in claim 1, which comprises
a) treating a tetraaroxyperylene-3,4,9,10-tetracarboxylic diimide of the general formula II 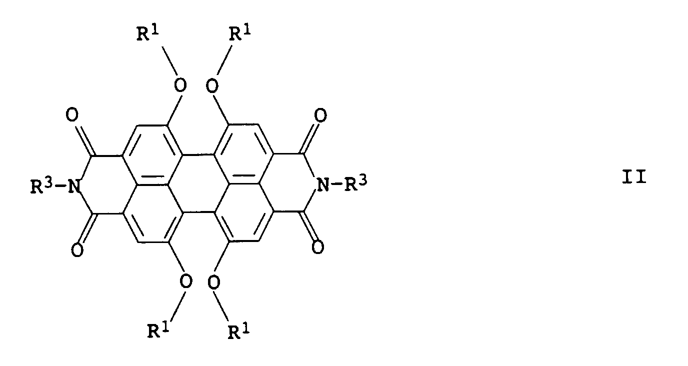 where the radicals R³ are C₁-C₃₀-alkyl radicals whose carbon chain may be interrupted by from 1 to 10 oxygen atoms in ether function, C₅-C₇-cycloalkyl or phenyl radicals which may each be substituted by up to 2 C₁-C₄-alkyl groups,
with a base in a polar, protic solvent, and
b) polycondensing the tetraaroxyperylene-3,4,9,10-tetracarboxylic dianhydride obtained in step a), of the general formula III 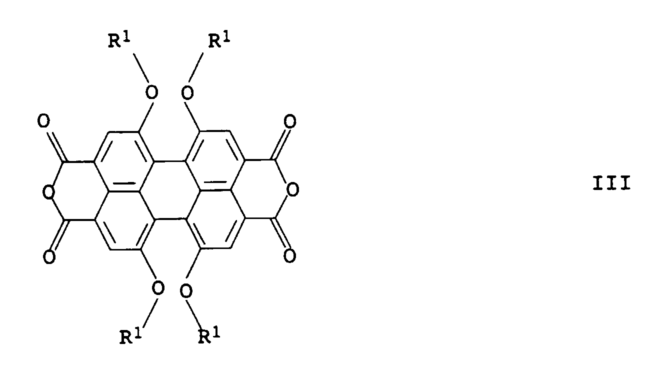 with a diamine of the general formula IV
H₂N-R²-NH₂ IV
in the presence of a polar solvent initially under acid and then under basic catalysis at from 170 to 200°C.

3. The use of tetraaroxyperylene-3,4,9,10-tetracarboxylic polyimides of the formula I as set forth in claim I for manufacturing dye lasers and light collectors and for pigmenting printing inks, paints and plastics.

4. Tetraaroxyperylene-3,4,9,10-tetracarboxylic dianhydrides of the general formula III 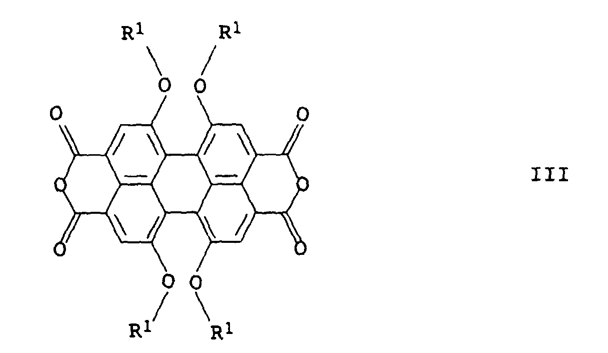 where the radicals R¹ denote identical or different aryl radicals which may be substituted by cyano, nitro, halogen, C₁-C₁₈-alkoxy, C₅-C₇-cycloalkyl and/or C₁-C₁₈-alkyl and which may each contain up to 24 carbon atoms,
and the corresponding tetraaroxyperylene-3,4,9,10-tetracarboxylic acids (IIIa).

## Revendications

1. Polyimides d'acide tétraaroxypérylène-3,4,9,10-tétracarboxylique de formule générale I 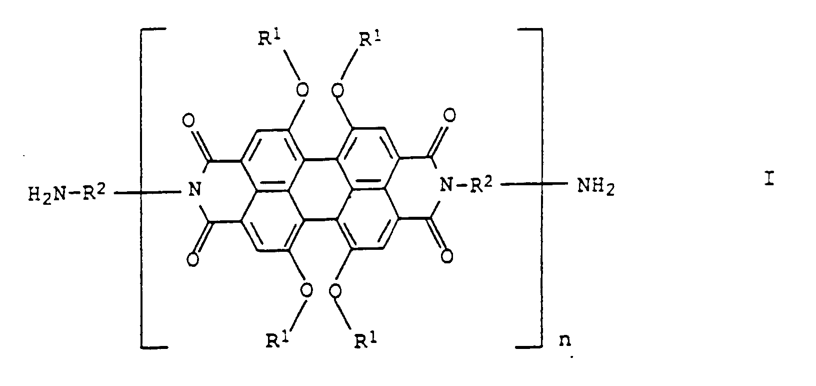 dans laquelle les variables ont les significations suivantes:
R¹ Restes aryle identiques ou différents qui peuvent être substitués par des groupements cyano, nitro, des atomes d'halogène ou des groupements alcoxy en C₁-C₁₈, cycloalkyle en C₅-C₇ et/ou alkyle en C₁-C₁₈ et qui peuvent contenir chacun jusqu'à 24 atomes de carbone;
R² Groupements alkylène en C₂-C₃₀, dont la chaîne carbonée peut être interrompue par 1 à 10 atomes d'oxygène en fonction éther ou par un groupement phénylène ou cyclohexylène, ou restes arylène en C₆-C₃₀ ou cyclobexylène éventuellement pontés par des groupements alkylène en C₁-C₁₀ ou des atomes d'oxygène;
n 2 à 100.

2. Procédé de préparation de polyimides d'acide tétraaroxypérylène-3,4,9,10-tétracarboxylique de formule I selon la revendication 1, caractérisé en ce que
a) on traite un diimide d'acide tétraaroxypérylène-3,4,9,10-tétracarboxylique de formule générale II 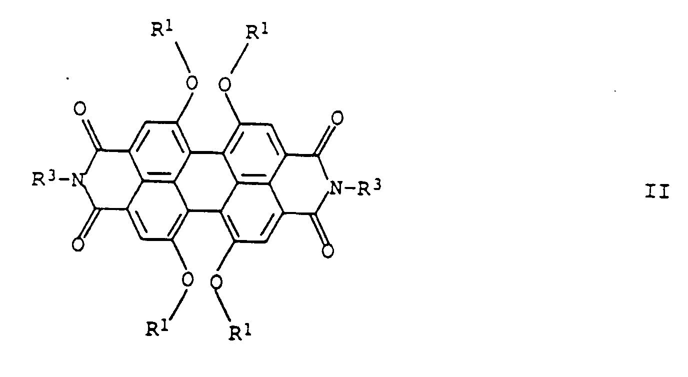 dans laquelle les restes R³ représentent des restes alkyle en C₁-C₃₀ dont la chaîne carbonée peut être interrompue par 1 à 10 atomes d'oxygène en fonction éther, des restes cycloalkyle en C₅-C₇ ou phényle qui peuvent être substitués chacun par 2 groupements alkyle en C₁-C₄ au maximum,
par une base dans un solvant protonique polaire, et
b) on polycondense le dianhydride d'acide tétraaroxypérylène-3,4,9,10-tétracarboxylique de formule générale III 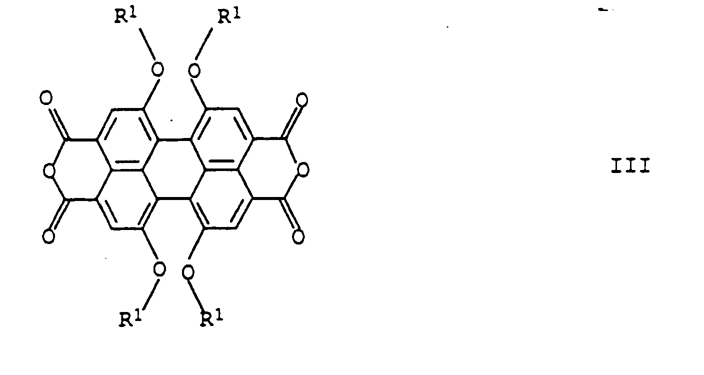 obtenu dans l'étape a) avec une diamine de formule générale IV
H₂N-R²-NH₂ IV
en présence d'un solvant polaire, d'abord sous catalyse acide, puis sous catalyse basique, a une température de 170 à 200°C.

3. Utilisation de polyimides d'acide tétraaroxypérylène-3,4,9,10-tétracarboxylique de formule I selon la revendication 1 pour la fabrication de lasers à colorants organiques accordables et de systèmes omnibus d'éclairage et pour la pigmentation d'encres d'imprimerie, de produits d'enduction et de matières plastiques.

4. Dianhydrides d'acide tétraaroxypérylène-3,4,9,10-tétracarboxylique de formule générale III 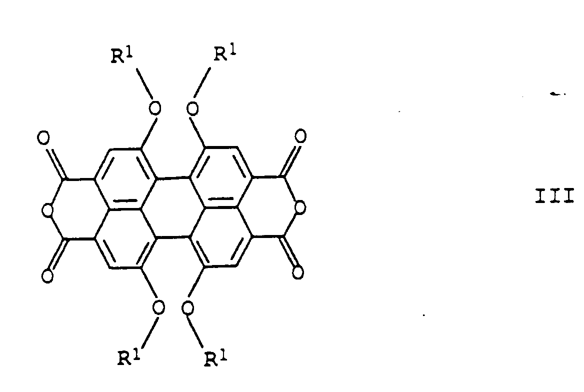 dans laquelle les restes R¹ représentent des restes aryle identiques ou différents qui peuvent être substitués par des groupements cyano, nitro, des atomes d'halogène ou des groupements alcoxy en C₁-C₁₈, cycloalkyle en C₅-C₇ et/ou alkyle en C₁-C₁₈ et qui peuvent contenir chacun jusqu'à 24 atomes de carbone,
et les acides tétraaroxypérylène-3,4,9,10-tétracarboxyliques (IIIa) correspondants.
